# EUROPÄISCHE PATENTSCHRIFT

(11) **EP 4 019 077 B1**
(45) Veröffentlichungstag und Bekanntmachung des Hinweises auf die Patenterteilung: **20.09.2023**
(21) Anmeldenummer: 20217020.5
(22) Anmeldetag: 23.12.2020
(51) Int. Cl.: A61N 1/04, A61N 1/375, A61N 1/40

(54) **ELEKTROTHERAPIEGERÄT**
ELECTROTHERAPY DEVICE
APPAREIL D'ÉLECTROTHÉRAPIE

(43) Veröffentlichungstag der Anmeldung: 29.06.2022
(73) Patentinhaber: Keytec GmbH, 86420 Diedorf (DE)
(72) Erfinder: BURR, Ulrich, 50374 Erftstadt (DE)
(74) Vertreter: Dantz, Jan Henning

(56) Entgegenhaltungen:
- WO-A1-02/085454
- WO-A1-2017/106878
- RU-A- 2004 136 418
- US-A1- 2016 059 023
- US-A1- 2019 022 400

## Beschreibung

Die Erfindung betrifft ein Elektrotherapiegerät gemäß dem Oberbegriff des geltenden Anspruchs 1.

Ein Elektrotherapiegerät gemäß dem Oberbegriff des geltenden Anspruchs 1 ist aus der RU 2004 136 418 A bekannt.

Die D2 offenbart eine Impedanzmessungsvariante für Defibrilliationsanwendungen.

Es ist Aufgabe der Erfindung, das bekannte Elektrotherapiegerät zu verbessern.

Gemäß einem Aspekt der Erfindung umfasst ein Elektrotherapiegerät zum Eintragen eines elektrischen Therapieimpulses in die Haut eines Patienten einen Schwingkreis mit einem Ausgabekondensator zum Eintragen des elektrischen Therapieimpulses in die Haut des Patienten und einer an den Ausgabekondensator angeschlossenen Schwingkreisspule, eine mit der Schwingkreisspule magnetisch gekoppelte Ladespule zum Laden der Schwingkreisspule, und eine Bestromungsvorrichtung zum Bestromen der Ladespule mit einem Ladestrom.

Erfindungsgemäß umfasst die Bestromungsvorrichtung Einstellmittel, mit der sich der Ladestrom in wenigstens drei verschiedenen Höhen einstellen lässt.

Der angegebenen Bestromungsvorrichtung liegt die Überlegung zugrunde, dass der Hautwiderstand eines Menschen nicht einheitlich ist. Der Hautwiderstand ist Teil des Körperwiderstandes einer zu behandelnden Person und kann zwischen den zwei Elektroden eines Ausgabekondensators Werte von einigen kΩ bis hin zu einigen 10kΩ annehmen.

Alle bisher bekannten Therapiegeräte können die Impulsstärke nur auf der Ausgangseite beeinflussen, durch Filter und/oder Dämpfungsglieder. Diese Möglichkeiten können auch im erfindungsgemäßen Elektrotherapiegerät erhalten bleiben.

Im Rahmen der vorliegenden Erfindung besteht nunmehr auch die Möglichkeit auch in der Aufladephase der Spule, also in der Zeit, wenn der Strom von der Stromquelle, vorzugsweise einem Akku, über eine Steuereinheit zur Spule und über die Haut des Anwenders/Patienten zurück zur gemeinsamen Masse fliest, die Impulsstärke patientenindividueller einzustellen.

In dieser Zeit bestimmt die Impedanz der Spule mit der parallelen Impedanz der Haut die Größe des Stromes. Die Zeitdauer des Stromes wird durch die Vorimpulszeit oder Ladezeit definiert und kann vom Therapeuten oder vom Programm eingestellt und vorgegeben werden.

Für empfindliche Menschen bestand bislang nur die Möglichkeit eine kurze Vorladezeit zu wählen, was zwangsläufig eine geringe Magnetisierung der Spule bedeutet und beim Abschalten der Vorbestromung zu einem kleinen und ineffizienten Therapieimpuls führt.

Die im Rahmen der vorliegenden Erfindung erreichte vorteilhafte variable Vorbestromung führt zu einer wahlweise langsameren Bestromung, die der Anwender/Patient als weniger unangenehm empfindet und gleichzeitig kann durch die Wahl einer geringfügig längeren Vorbestromungszeit / Aufladezeit die gleiche Magnetisierung der Spule erreicht und somit ein effektiverer Therapieimpuls erzeugt werden.

Der Therapieimpuls ist dabei vorzugsweise nur von der Magnetisierung, also von der gespeicherten magnetischen Energie, abhängig. Dieser wird durch die variable Vorbestromung nicht oder nur in geringem Umfang beeinflusst.

Wird der Therapieimpuls dabei durch Laden einer Spule mit einem Ladestrom und durch entladen der Spule über einen Schwingkreis mit dem Ausgabekondensator erzeugt, so hängt die Stärke des Therapieimpulses auch vom Hautwiderstand der zu behandelnden Person ab, auf die der Ausgabekondensator aufgelegt ist. Weil eine geladene Spule beim Abschalten bekanntermaßen ihr Magnetfeld so schnell wie möglich abbaut, wird eine elektrische Spannung des Ausgabekondensators umso höher sein, je höher der Körperwiderstand zur behandelnden Person ist. Daher können Menschen mit einem hohen Hautwiderstand den Vorimpuls und den Therapieimpuls als äußerst unangenehm empfinden.

Dabei sind der Vorimpuls und Therapieimpuls vorzugsweise aufeinander folgende Zeitabläufe. Durch das Aufladen des magnetischen Speichers wird der Vorimpuls generiert, während durch das Entladen des magnetischen Speichers der Therapieimpuls. In beiden Fällen können unangenehme Effekte durch Strom und Spannung entstehen.

Dieser unangenehmen Empfindung wurde in dem eingangs genannten Elektrotherapiegerät versucht, durch Frequenz- und/oder Amplitudenmodulation als auch durch eine ausreichende Dämpfung entgegenzuwirken. Allerdings sind diese Mittel technisch suboptimal, denn der Therapiepuls wird anfänglich aufgrund der geladenen Spule im Zusammenhang mit dem vergleichsweise hohen Hautwiderstand einiger Patienten immer mit einem vergleichsweise hohen Ausschlag beginnen. Unabhängig davon wird durch Modulation und/oder anderweitige Veränderung des Therapiepulses elektrische Energie vernichtet.

Mit dem erfindungsgemäßen Einstellmittel, mit der sich der Ladestrom in wenigstens vier verschiedenen Höhen einstellen lässt, die Spule mehr oder weniger schnell aufzuladen, lässt sich auf die unterschiedlich hohen Körperwiderstände der zu behandelnden Personen an der Ursache für das unangenehme Gefühl einiger weniger Personen ansetzen und direkt auf ihre Empfindung reagieren. Auf diese Weise lässt sich ohne Vernichtung elektrischer Energie direkt auf das Schmerzempfinden der jeweiligen zu behandelnden Personen Einfluss nehmen und das Therapieverfahren mit dem angegebenen Elektrotherapiegerät an die Bedürfnisse der zu behandelnden Personen anpassen.

Weiterhin ist es durch einen erhöhten schaltungstechnischen Aufbau auch möglich eine stufenlose Steuerung der Vorbestromung zu realisieren. Um eine hohe Reproduzierbarkeit des Stromflusses zu erreichen, z.B. als Ausgleich von Bauelemente-Toleranzen und/oder Kanalwiderständen, sollte dies mit einer Rückkopplung zum Steuertransistor, also als Regelung erfolgen. Durch Nutzung einer Mehrstufigkeit der vorliegend realisierten Steuerung kann dieser erhöhte Aufwand vermieden werden.

In einer Weiterbildung des angegebenen Elektrotherapiegerätes umfasst die Bestromungsvorrichtung eine gesteuerte Spannungsquelle zur Ausgabe einer gesteuerten Spannung und einen Stromverstärker zur Ausgabe des Ladestromes basierend auf der gesteuerten Spannung. Auf diese Weise lässt sich die Einstellung der Höhe des Ladestromes vom Ein- und Ausschalten des Ladestromes technisch trennen, so dass sich beim Einschalten des Ladestromes gesichert ein bestimmter Ladestrom ausgeben lässt.

Zwar kann die gesteuerte Spannungsquelle grundsätzlich gemeinsam mit dem Stromverstärker einstellbar sein, ist der Stromverstärker allerdings unabhängig von der gesteuerten Spannungsquelle einschaltbar, lässt sich die von der gesteuerten Spannungsquelle ausgegebene gesteuerte Spannung vorab prüfen, um beispielsweise das angegebene Elektrotherapiegerät auf Fehleinstellungen zu überprüfen.

In einer der besonderen Weiterbildung des angegebenen Elektrotherapiegerätes umfasst der Stromverstärker zwei zu einer Darlington-Schaltung zusammengesetzte Transistoren mit denen sich auf kleinstem Bauraum Verstärkungsfaktoren von bis zu 50.000 erreichen lassen. Wird die Darlington-Schaltung aus zwei komplementären Transistoren nach Sziklai zusammengesetzt, kann die Einschaltspannung deutlich reduziert und das angegebene Elektrotherapiegerät in herkömmliche informationstechnische Datenverarbeitungsgeräte integriert werden.

Die Verwendung dieser Schaltungsart ist mit zumindest zwei Vorteilen verbunden. Einerseits wird ein schnelles Schalten, vergleichsweise großer Ströme mit kleinen Transistoren, durch Verwendung der angepassten Darlington Struktur erreicht.

Andererseits ergibt sich eine gewisse Schaltlogik durch diese Struktur, da der verwendete spannungsfeste Kleinleistungstransistor vorzugsweise als ein PNP-Transistor ausgebildet sein kann als ein sogenannter High-Side-Schalter und eine Pegelwandlung, vorzugsweise durch einen NPN-Transistor, zur Erhaltung der Logik sinnvoll ist. Dabei entspricht der Schaltungszustand "High" einem Stromfluss und "Low" einem gesperrten Zustand.

In einer anderen Weiterbildung des angegebenen Elektrotherapiegerätes ist die Spannungsquelle eingerichtet, die gesteuerte Spannung in diskreten Stufen zu erzeugen. Die einzelnen Stufen der gesteuerten Spannung lassen sich für spezielle Anwendungsfälle definieren, so dass sich das angegebene Elektrotheraphiegerät in besonders einfacher Weise bedienen lässt.

In einer zusätzlichen Weiterbildung umfasst die Spannungsquelle des angegebenen Elektrotherapiegerätes zur Erzeugung der diskreten Stufen eine Logikschaltung. Mit der Logikschaltung lässt sich die gesteuerte Spannungsquelle über eine Schnittstelle eines Mikrokontrollers oder Computers ansteuern und bedienen.

In einer besonderen Weiterbildung umfasst die Logikschaltung des angegebenen Elektrotherapiegerätes einen ersten Schalteingang und einen zweiten Schalteingang, die je einen eigenen Schalter und gemeinsames NOR-Gatter ansteuern. Auf diese Weise lassen sich mit den beiden Schalteingängen vier verschiedene diskrete Spannungsstufen erzeugen.

Besonders bevorzugt ist die gesteuerte Spannungsquelle eingerichtet, als erste Stufe eine maximale Spannung und als vierte Stufe die Hälfte der maximalen Spannung auszugegeben. Dabei sind eine zweite Stufe und eine dritte Stufe im Wesentlichen zwischen der ersten Stufe und der vierten Stufe gleichverteilt Gemäß einem weiteren Aspekt der Erfindung umfasst ein Computerprogramm Programmcodemittel, um alle Schritte des angegebenen Verfahrens durchzuführen, wenn das Computerprogramm auf einem elektronischen Gerät oder einer der angegebenen Vorrichtungen ausgeführt wird.

Gemäß einem weiteren Aspekt der Erfindung sind die Programmcodemittel speziell dafür vorgesehen, die richtige logische Konfiguration und den notwendigen zeitlichen Ablauf der Steuervorrichtung zu gewährleisten.

Erfindungsgemäß ist das Elektrotherapiegerät als Handgerät, auch "hand-held" genannt, ausgebildet.

Weiter bevorzugt kann das Elektrotherapiegerät, insbesondere in der Ausgestaltung als Handgerät, batterie- oder akku-betrieben ausgebildet sein.

Die oben beschriebenen Eigenschaften, Merkmale und Vorteile dieser Erfindung sowie die Art und Weise wie diese erreicht werden, werden verständlicher im Zusammenhang mit der folgenden Beschreibung der Ausführungsbeispiele, die im Zusammenhang mit der Zeichnung näher erläutert werden. Es zeigen:
Fig. 1 eine schematische Darstellung eines Elektrotherapiegerätes,
Fig. 2 eine schematische Darstellung einer gesteuerten Spannungsquelle des Elektrotherapiegerätes der Fig. 1,

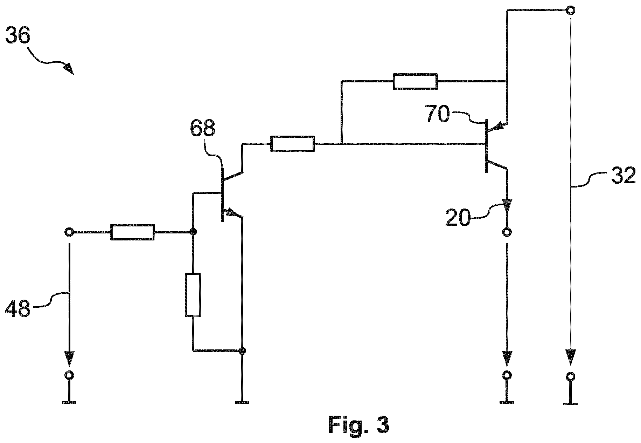

Fig. 3 eine schematische Darstellung eines gesteuerten Stromverstärkers des Elektrotherapiegerätes der Fig. 1, und

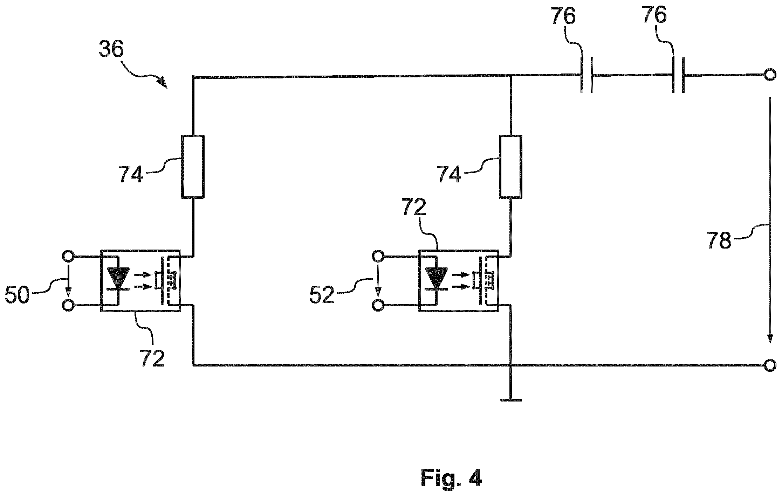

Fig. 4 eine schematische Darstellung eines Dämpfungsschaltkreises des Elektrotherapiegerätes der Fig. 1.

In den Figuren werden gleiche technische Elemente mit gleichen Bezugszeichen versehen und nur einmal beschrieben. Die Figuren sind rein schematisch und geben vor allem nicht die tatsächlichen geometrischen Verhältnisse wieder.

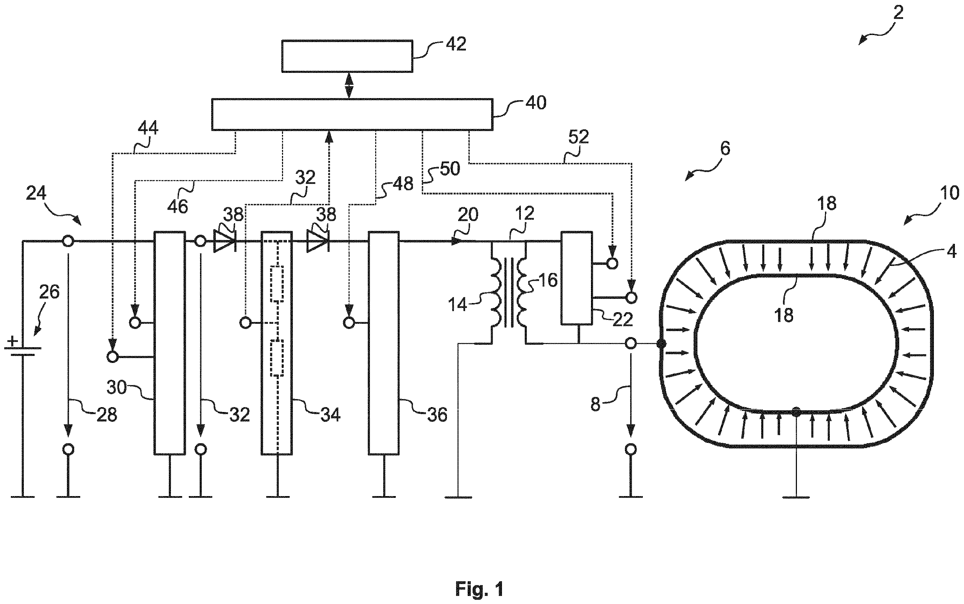

Es wird auf Fig. 1 Bezug genommen, die eine schematische Darstellung eines Elektrotherapiegerätes 2 zum Eintragen eines elektrischen Therapieimpulses 4 in die Haut eines nicht weiter gezeigten Patienten zeigt. Der Therapiepuls 4 wird in Form eines elektrischen Feldes abgegeben, welches in Fig. 1 durch seine Feldlinien angedeutet ist.

Zur Erzeugung der Therapiepulses 4 in Form des elektrischen Feldes umfasst das Elektrotherapiegerät 2 eine Pulsquelle 6 zur Ausgabe einer Pulsspannung 8. In der vorliegenden Ausführung umfasst die Pulsquelle 6 aus einem Schwingkreis aus einem den Therapiepuls 4 ausgebenden Ausgabekondensator 10 und einem Transformator 12, der aus einer an den Ausgabekondensator 10 angeschlossenen Sekundärspule 16 und einer die Sekundärspule 16 speisenden Primärspule 14 gebildet ist. Der Ausgabekondensator 10 kann grundsätzlich beliebig ausgebildet sein, ist aber in der vorliegenden Ausführung durch zwei konzentrisch zueinander angeordnete Elektroden 18 gebildet.

In dem Transformator 12 wird die Sekundärspule 16 über die Primärspule 14 geladen, weshalb die Sekundärspule 16 als Teil des Schwingkreises nachstehend Schwingkreisspule 16 und die Primärspule 14 als die die Schwingkreisspule 16 ladende Ladespule 14 bezeichnet wird.

Zur Verwendung des Elektrotherapiegerätes 2 wird der Ausgabekondensator 10 auf die Haut des Patienten gelegt. Anschließend wird die Schwingkreisspule 16 durch Bestromen der Ladespule 14 mit einem Ladestrom 20 magnetisch geladen. Gleichzeitig fließt ein Teil des Ladestroms über die Sekundärspule 16 zu einer Elektrode 18a des Ausgangskondensators 10 und zur Haut des Anwenders / Patienten. Dieser Ladestrom 20, der sich in den die Spule magnetisierenden Teil und den Patiententeil verzweigt, ist im Rahmen der vorliegenden Erfindung in seiner Stärke erstmalig steuerbar.

Durch Unterbrechen des Ladestromes 20 entlädt sich die sich Schwingkreisspule 16 über den Ausgabekondensator 10, der wiederum den Therapieimpuls 4 in Form des elektrischen Feldes erregt.

Das elektrische Feld des Therapiepulses 4 ist abhängig vom elektrischen Hautwiderstand des Patienten, auf den der Ausgabekondensator 10 aufgelegt ist. Je höher der elektrische Hautwiderstand des Patienten ist, desto stärker ist das elektrische Feld, weil die Schwingkreisspule 16 bestrebt ist, ihr magnetisches Feld so schnell wie möglich abzubauen. An Patienten mit einem vergleichsweise hohen elektrischen Hautwiderstand könnte daher ein vergleichsweise großes elektrisches Feld als Therapieimpuls 4 angelegt werden, was für den Patienten als unangenehm bis sogar schmerzhaft empfunden werden kann.

Zwar könnte an die Schwingkreisspule 16 ein Dämpfungsschaltkreis 22 angeschlossen werden, der die Spannung an der Schwingkreisspule 16 und damit das elektrische Feld des Therapieimpulses 4 dämpft, dem anfänglich hohen Spannungsaufbau in der Schwingkreisspule 16 kann auf diese Weise allerdings nur begrenzt begegnet werden. Zwar könnte durch weitere Maßnahmen an der Schwingkreisspule 16, wie beispielsweise einer Modulation der Spannung an der Schwingkreisspule 16 weiter versucht werden, auf den zeitlichen Verlauf des elektrischen Feldes des Therapieimpulses 4 Einfluss zu nehmen. Alle diese Maßnahmen sind jedoch zur Begrenzung des anfänglich hohen Spannungsaufbaus aufgrund des Abbaus des magnetischen Feldes in der Schwingkreisspule 16 ungeeignet.

Mit der vorliegenden Ausführung wird vorgeschlagen, den Ladestrom 20 mit einer Bestromungsvorrichtung 24 einzustellen. Auf diese Weise lässt sich die Höhe des in der Schwingkreisspule 16 aufgebauten magnetischen Feldes steuern und die Höhe des elektrischen Feldes des Therapieimpulses 4 auch in der Anfangsphase wirksam steuern.

Die Bestromungsvorrichtung 24 weist eine Quellspannungsquelle 26 aus der eine Quellspannung 28 entnehmbar ist. Die Quellspannung 28 wird in einer Spannungssteuerschaltung 30 in eine gesteuerte Spannung 32 gewandelt, die mit einem hier beispielhaft als Spannungsteiler ausgebildeten Spannungsmessgerät 34 überprüfbar ist. Die Quellspannungsquelle 26 bildet somit gemeinsam mit der Spannungssteuerschaltung 30 eine gesteuerte Spannungsquelle. Ein Stromverstärker 36 konvertiert abschließend aus der gesteuerten Spannung 32 den Ladestrom 20. Die Bestromungsvorrichtung 24 umfasst Rückschlagdioden 38, um ein Spannungsrückschlag aus der Ladespule 14 zurück in die restliche Schaltung zu vermeiden.

Angesteuert wird die Bestromungsvorrichtung 24 von einer Steuervorrichtung 40, an die Ein- und Ausgabemittel 42 zur Bedienung angeschlossen sein können. Die Steuervorrichtung 40 steuert in der vorliegenden Ausführung mit einem ersten Spannungssteuersignal 44 und einem zweiten Spannungssteuersignal 46 in einer noch zu beschreibenden Weise die Spannungssteuerschaltung 30 zur Erzeugung der gesteuerten Spannung 32 an. Mit einem Einschaltsignal 48 kann der Stromverstärker 36 über die Steuervorrichtung 40 gesteuert werden. Schließlich lässt sich der Dämpfungsschaltkreis 22 mit einem ersten Dämpfungssteuersignal 50 und einem zweiten Dämpfungssteuersignal 52 über die Steuervorrichtung 40 ansteuern.

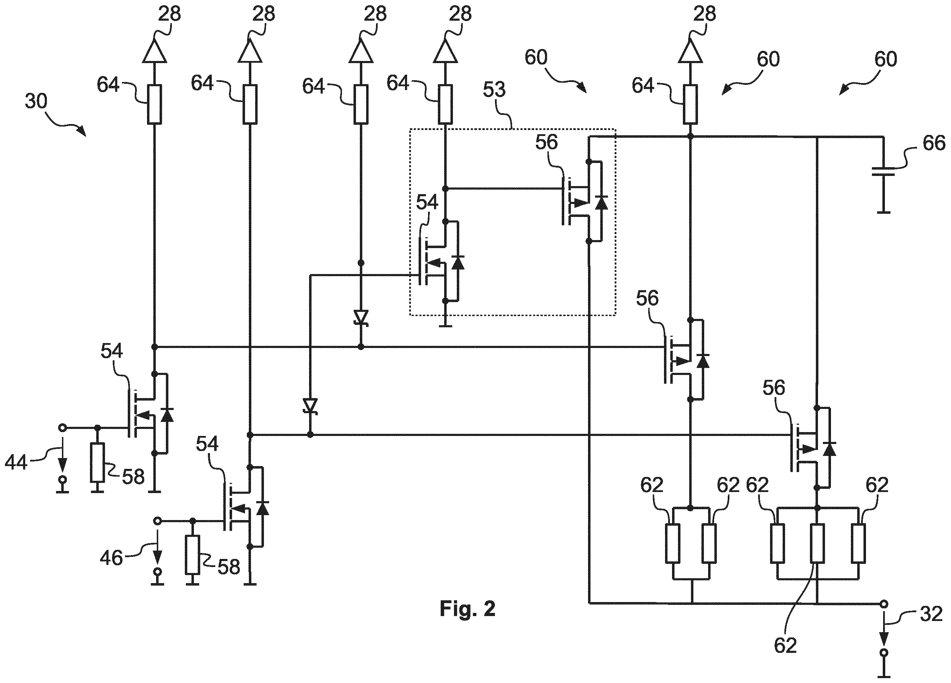

Mit Bezug auf Fig. 2 wird nachstehend die Spannungssteuerschaltung 30 näher beschrieben.

Die beiden Spannungssteuersignale 44, 46 steuern je einen drahtbruchsicheren Schalter 53 aus Komplementärschaltelementen an, die in der vorliegenden Ausführung als eine Reihenschaltung aus je einem n-Kanal-Feldeffekttransistoren 54 und einem p-Kanal-Feldeffekttransistor 56 ausgebildet sind. Der jeweilige Schalteingang, an den die beiden Spannungssteuersignale 44, 46 gelegt sind, ist mit je einem Vorwidertand 58 gesichert.

Mit einem Potential zwischen den Komplementärschaltelementen der durch die Spannungssteuersignale 44, 46 angesteuerten drahtbruchsicheren Schalter 53 wird zum Ansteuern eines dritten drahtbruchsicheren Schalters 53 verwendet. Auf diese Weise ist ein NOR-Gatter aus drei n-Kanal-Feldeffekttransistoren 53 und einem p-Kanal-Feldeffekttransistor 56 geschaffen, das schaltet, wenn keines der beiden Spannungssteuersignale 44, 46 eingeschalten ist.

Jeder drahtbruchsichere Schalter 53 kann einen Strompfad 60 zwischen der Potenzialseite der Quellspannung 28 und der Potenzialseite der zu erzeugenden gesteuerten Spannung 32 individuell schließen. Ferner weist jeder Strompfad 60 einen unterschiedlichen Widerstandswert auf, was in Fig. 2 durch eine unterschiedliche Anzahl an parallel geschalteten Steuerwiderständen 62 in den einzelnen Strompfaden 60 angedeutet ist. Auf diese Weise lassen sich mit der Spannungssteuerschaltung 30 4 verschiedene Spannungen als gesteuerte Spannung 32 erzeugen.

Die Spannungssteuerschaltung 30 weist ferner noch Schutzwiderstände 64 und Schutzkapazitäten 66 auf, die allerdings zum Verständnis der vorliegenden Ausführung nicht weiter notwendig ist.

Die von der Spannungssteuerschaltung 30 ausgegebene gesteuerte Spannung 32 wird abschließend an den Stromverstärker 36 ausgegeben, der wiederum den Ladestrom 20 erzeugt. Aufgabe des Stromverstärkers 36 ist es schnell einen von der Spannungssteuerschaltung 30 begrenzten aber möglichst hohen Ausgangsstrom als Ladestrom 20 zu generieren.

Hierzu können grundsätzlich beliebige Stromverstärker-Typen verwendet werden. In der vorliegenden Ausführung ist der Stromverstärker 36 als komplementäre Darlington-Schaltung, also als sogenannten Sziklai-Paar mit einem durch einen NPN-Bipolartransistor 68 angesteuerten PNP-Bipolartransistor 70 realisiert, wobei der NPN-Bipolartransistor 68 zum Einschalten des Stromverstärkers 36 mit dem oben erwähnten Einschaltsignal 48 angesteuert wird. Die Dimensionierung einer Darlington-Schaltung, ob komplementär oder mit gleichen Transistoren ist an sich bekannt. Im vorliegenden Fall ist die Auswahl der Transistoren funktional begründet, wie sich bereits aus der vorhergehenden Beschreibung ergibt.

Im Betrieb der Bestromungsvorrichtung 24 stellt der Benutzer zunächst mit der Steuervorrichtung 40 beispielsweise über die Benutzerschnittstelle 42 die Spannungssteuersignale 44, 46 derart ein, dass die Ladespule 14 mit einem für einen gewünschten Therapieimpuls 4 notwendigen Ladestrom 20 geladen wird. Mit den beiden Spannungssteuersignalen 44, 46 lassen sich der Ladestrom 20 in vier diskreten Stufen erzeugen. Diese vier Stufen können nach verschiedenen Strategien verteilt werden, beispielsweise in dem die Stufen zwischen 50% und 100% des maximal erzeugbaren Ladestromes gleich verteilt werden. Somit wäre die erste Stufe bei 3/6-tel des maximal erzeugbaren Ladestromes, die zweite Stufe bei 4/6-tel des maximal erzeugbaren Ladestromes, die dritte Stufe bei 5/6-tel des maximal erzeugbaren Ladestromes und die vierte Stufe bei 6/6-tel des maximal erzeugbaren Ladestromes. In Prozent ausgedrückt sind dies entsprechend 50% des maximal erzeugbaren Ladestromes, zirka 66% des maximal erzeugbaren Ladestromes, zirka 85% des maximal erzeugbaren Ladestromes und 100% des maximal erzeugbaren Ladestromes.

Hat der Benutzer die Spannungssteuersignale 44, 46 definiert und die Spannungssteuerschaltung 30 entsprechend eingestellt, wird der Ladestrom 20 durch Einschalten des Stromverstärkers 36 eingeschaltet, bis die Ladespule 14 geladen ist. Anschließend wird die Ladespule 14 ausgeschaltet und der Therapieimpuls 4 in der oben beschriebenen Weise erzeugt.

Dieser kann mit dem an sich bekannten Dämpfungsschaltkreis 22 optional gedämpft werden.

Der Dämpfungsschaltkreis 22 dieser Ausführung weist parallel zur Schwingkreisspule 16 geschaltete und individuell über Optokoppler 72 ansteuerbare Dämpfungswiderstände 74 auf, die mittels Gleichspannungsfilterkapazitäten 76 von der Schwingkreisspule 16 entkoppelt sind und dämpft die von der Schwingkreisspule 16 ausgegebene Spannung 78.

## Patentansprüche

1. Elektrotherapiegerät (2) als Handgerät zum Eintragen eines elektrischen Therapieimpulses (4) in die Haut eines Patienten, umfassend:
- einen Schwingkreis mit einem Ausgabekondensator (10) zum Eintragen des elektrischen Therapieimpulses (4) in die Haut des Patienten und einer an den Ausgabekondensator (10) angeschlossenen Schwingkreisspule (16),
- eine mit der Schwingkreisspule (16) magnetisch gekoppelte Ladespule (14) zum Laden der Schwingkreisspule (16), und
- eine Bestromungsvorrichtung (24) zum Bestromen der Ladespule (14) mit einem Ladestrom (20),
**dadurch gekennzeichnet, dass** die Bestromungsvorrichtung (24) Einstellmittel (30) umfasst, mit der sich der Ladestrom (20) in wenigstens drei, insbesondere wenigstens vier, verschiedenen Höhen einstellen lässt.

2. Elektrotherapiegerät (2) nach Anspruch 1, **dadurch gekennzeichnet, dass** die Bestromungsvorrichtung (24) eine gesteuerte Spannungsquelle (26, 30) zur Ausgabe einer gesteuerten Spannung (32) und einen Stromverstärker (36) zur Ausgabe des Ladestromes (20) basierend auf der gesteuerten Spannung (32) umfasst.

3. Elektrotherapiegerät (2) nach Anspruch 2, **dadurch gekennzeichnet, dass** der Stromverstärker (36) unabhängig von der gesteuerten Spannungsquelle (26, 30) einschaltbar ist.

4. Elektrotherapiegerät (2) nach Anspruch 2 oder 3, **dadurch gekennzeichnet, dass** der Stromverstärker (36) zwei zu einer Darlington-Schaltung zusammengesetzte, vorzugsweise komplementäre, Transistoren (68, 70) umfasst.

5. Elektrotherapiegerät (2) nach einem der vorstehenden Ansprüche 2 bis 4, **dadurch gekennzeichnet, dass** die gesteuerte Spannungsquelle (26, 30) eingerichtet ist, die gesteuerte Spannung (32) in diskreten Stufen zu erzeugen.

6. Elektrotherapiegerät (2) nach Anspruch 5, **dadurch gekennzeichnet, dass** die gesteuerte Spannungsquelle (26, 30) zur Erzeugung der diskreten Stufen eine Logikschaltung (30) umfasst.

7. Elektrotherapiegerät (2) nach Anspruch 6, **dadurch gekennzeichnet, dass** die Logikschaltung (30) einen ersten Schalteingang (44) und einen zweiten Schalteingang (46) umfasst, die je einen eigenen Schalter (53) und gemeinsames NOR-Gatter ansteuern.

8. Elektrotherapiegerät (2) nach einem der Ansprüche 5 bis 7, **dadurch gekennzeichnet, dass** die gesteuerte Spannungsquelle (26, 30) eingerichtet ist, als erste Stufe eine maximale Spannung und als vierte Stufe die Hälfte der maximalen Spannung auszugegeben, und wobei die zweite Stufe und die dritte Stufe im Wesentlichen zwischen der ersten Stufe und der vierten Stufe gleichverteilt sind.

9. Elektrotherapiegerät nach einem der vorhergehenden Ansprüche, umfassend eine Steuervorrichtung (40) zum Erzeugen eines Therapieimpulses (4) mit der Spule (16) und dem den Therapieimpuls (4) in die Haut eines Patienten eintragenden Ausgabekondensator (10) enthaltenden Schwingkreises, wobei die Steuervorrichtung (40) ausgebildet ist zum:
- Einstellen eines Ladestrom (20) in wenigstens einer von vier verschiedenen Höhen,
- Laden der Spule (16) basierend auf dem Ladestrom, und
- Entladen der Spule (16) über den Ausgabekondensator (10) zur Erzeugung des Therapieimpulses (4) in Form eines elektrischen Feldes.

## Claims

1. Electrotherapy device (2) as a hand-held device for applying an electrical therapy pulse (4) to the skin of a patient, comprising:
- an oscillating circuit having an output capacitor (10) for applying the electrical therapy pulse (4) to the skin of a patient and an oscillating circuit coil (16) connected to the output capacitor (10),
- a charging coil (14) magnetically coupled to the oscillating circuit coil (16) for charging the oscillating circuit coil (16), and
- an energizing device (24) for energizing the charging coil (14) with a charging current (20),
**characterized in that** the energizing device (24) comprises adjusting means (30) with which the charging current (20) can be adjusted in at least three, in particular at least four, different levels.

2. Electrotherapy device (2) according to claim 1, **characterized in that** the energizing device (24) comprises a controlled voltage source (26, 30) for outputting a controlled voltage (32) and a current amplifier (36) for outputting the charging current (20) based on the controlled voltage (32).

3. Electrotherapy device (2) according to claim 2, **characterized in that** the current amplifier (36) is switchable on independently of the controlled voltage source (26, 30).

4. Electrotherapy device (2) according to claim 2 or 3, **characterized in that** the current amplifier (36) comprises two, preferably complementary, transistors (68, 70) assembled to form a Darlington circuit.

5. Electrotherapy device (2) according to one of the preceding claims 2 to 4, **characterized in that** the controlled voltage source (26, 30) is arranged to generate the controlled voltage (32) in discrete stages.

6. Electrotherapy device (2) according to claim 5, **characterized in that** the controlled voltage source (26, 30) comprises a logic circuit (30) for generating the discrete stages.

7. Electrotherapy device (2) according to claim 6, **characterized in that** the logic circuit (30) comprises a first switching input (44) and a second switching input (46), each driving a dedicated switch (53) and common NOR gate.

8. Electrotherapy device (2) according to one of claims 5 to 7, **characterized in that** the controlled voltage source (26, 30) is arranged to output as first stage a maximum voltage and as fourth stage half of the maximum voltage, and wherein the second stage and the third stage are substantially equally distributed between the first stage and the fourth stage.

9. Electrotherapy device according to one of the preceding claims, comprising a control device (40) for generating a therapy pulse (4) with the coil (16) and the oscillating circuit containing the output capacitor (10) applying the therapy pulse (4) to the skin of a patient, wherein the control device (40) is adapted to:
- setting a charging current (20) at at least one of four different levels,
- charging the coil (16) based on the charging current, and
- discharging the coil (16) via the output capacitor (10) to generate the therapy pulse (4) in the form of an electric field.

## Revendications

1. Appareil d'électrothérapie (2) conçu comme un appareil manuel pour appliquer une impulsion électrique thérapeutique (4) dans la peau d'un patient, comprenant :
- un circuit oscillant avec un condensateur de sortie (10) pour appliquer l'impulsion électrique thérapeutique (4) dans la peau du patient et une bobine de circuit oscillant (16) raccordée au condensateur de sortie (10),
- une bobine de charge (14) en couplage magnétique avec la bobine de circuit oscillant (16) pour charger la bobine de circuit oscillant (16) et
- un dispositif d'arrivée de courant (24) pour amener un courant de charge (20) à la bobine de charge (14),
**caractérisé en ce que** le dispositif d'arrivée de courant (24) comprend des moyens de réglage (30) avec lesquels le courant de charge (20) peut être réglé à au moins trois, en particulier au moins quatre, niveaux différents.

2. Appareil d'électrothérapie (2) selon la revendication 1, **caractérisé en ce que** le dispositif d'arrivée de courant (24) comprend une source de tension régulée (26, 30) destinée à produire une tension régulée (32) et un amplificateur de courant (36) destiné à émettre le courant de charge (20) à partir de la tension régulée (32).

3. Appareil d'électrothérapie (2) selon la revendication 2, **caractérisé en ce que** l'amplificateur de courant (36) peut être activé indépendamment de la source de tension régulée (26, 30).

4. Appareil d'électrothérapie (2) selon la revendication 2 ou 3, **caractérisé en ce que** l'amplificateur de courant (36) comprend deux transistors (68, 70), de préférence complémentaires, regroupés dans un circuit de Darlington.

5. Appareil d'électrothérapie (2) selon l'une des revendications 2 à 4, **caractérisé en ce que** la source de tension régulée (26, 30) est configurée pour produire la tension régulée (32) par niveaux discrets.

6. Appareil d'électrothérapie (2) selon la revendication 5, **caractérisé en ce que** la source de tension régulée (26, 30) comprend un circuit logique (30) pour produire les niveaux discrets.

7. Appareil d'électrothérapie (2) selon la revendication 6, **caractérisé en ce que** le circuit logique (30) comprend une première entrée de commutation (44) et une deuxième entrée de commutation (46) qui contrôlent chacune son propre commutateur (53) et une grille NON-OU commune.

8. Appareil d'électrothérapie (2) selon l'une des revendications 5 à 7, **caractérisé en ce que** la source de tension régulée (26, 30) est configurée pour émettre au premier niveau une tension maximale et au quatrième niveau la moitié de la tension maximale, le deuxième niveau et le troisième niveau étant répartis à intervalles sensiblement égaux entre le premier niveau et le quatrième.

9. Appareil d'électrothérapie selon l'une des revendications précédentes, comprenant un dispositif de commande (40) destiné à produire une impulsion thérapeutique (4) avec la bobine (16) et le circuit oscillant comprenant le condensateur de sortie (10) qui applique l'impulsion thérapeutique (4) dans la peau d'un patient, lequel dispositif de commande (40) est conçu pour :
- régler un courant de charge (20) à au moins un niveau parmi quatre différents,
- charger la bobine (16) à partir du courant de charge et
- décharger la bobine (16) via le condensateur de sortie (10) pour produire l'impulsion thérapeutique (4) sous la forme d'un champ électrique.
